(19) Europäisches Patentamt
European Patent Office
Office européen des brevets

(11) Publication number: **0 137 692**
**A2**

(12) **EUROPEAN PATENT APPLICATION**

(21) Application number: 84305861.1

(22) Date of filing: 28.08.84

(51) Int. Cl.[4]: C 07 G 11/00
C 12 P 1/06, C 12 P 19/02
C 07 H 7/00, A 61 K 35/66
//(C12P1/06, C12R1:55)

(30) Priority: 30.08.83 JP 157214/83

(43) Date of publication of application:
17.04.85 Bulletin 85/16

(84) Designated Contracting States:
AT BE CH DE FR GB IT LI LU NL SE

(71) Applicant: Arai, Tadashi
50-6 Nogata 6-chome
Nakano-Ku Tokyo(JP)

(72) Inventor: Arai, Tadashi
50-6 Nogata 6-chome
Nakano-Ku Tokyo(JP)

(74) Representative: Marsh, Roy David et al,
Brewer & Son 5-9, Quality Court Chancery Lane
London WC2A 1HT(GB)

(54) **New antibiotic, neocopiamycin A and the production thereof.**

(57) A new antibiotic, designated as neocopiamycin A, is useful as antifungal agent. Neocopiamycin A is fermentatively produced as a minor component, in addition to copiamycin, by a microorganism *Streptomyces hygroscopicus* var. *crystallogenes* (FERM BP-576) which is known to produce the known antibiotic copiamycin.

EP 0 137 692 A2

Croydon Printing Company Ltd.

NEW ANTIBIOTIC, NEOCOPIAMYCIN A AND THE PRODUCTION THEREOF.

This invention relates to a new and useful antibiotic and the production thereof. More particularly, this invention relates to a new antibiotic designated as neocopiamycin A which is useful as a new antifungal agent and which is produced fermentatively by the cultivation of a microorganism, Streptomyces hygroscopicus var. crystallogenes.

This invention further relates to a process for the production of neocopiamycin A and uses of neocopiamycin A.

It is known that a known antibiotic, copiamycin, is produced fermentatively by the cultivation of such a strain of actinomycetes which was initially designated as Strain No. 1898 and then as Streptomyces hygroscopicus var. crystallogenes (identifiable as ATCC 19040)(see Japanese patent publication No. 15519/66; "Journal of Antibiotics" 18, 2, pages 63-67 (1965) and U.K. patent No. 1,124,093).

As a result of recent researches, it has been found that Streptomyces hygroscopicus var. crystallogenes produces several minor components, in addition to the main product, copiamycin which is a macrocyclic lactone antifungal antibiotic. One of these minor components

has now been isolated and designated as neocopiamycin A. Neocopiamycin A has now been proved to be more active against a number of fungi than copiamycin but less toxic than copiamycin.

There has been increasing demand for therapeutic chemical agents for the control and prevention of fungal diseases which affect plants and animals including human beings. It is therefore an object of the invention to provide a new antibiotic which is useful for the control of the growth of fungi. Other objects of invention will be apparent from the following descriptions and claims.

According to an aspect of this invention, therefore, there is provided a new antibiotic, neocopiamycin A, which is in the form of a colorless, hygroscopic substance having a melting point of 134-136°C (dec.) and giving a molecular formula $C_{53}H_{93}O_{17}N_3$; which is soluble in a lower alkanol including methanol, ethanol and n-butanol, pyridine, dimethylformamide and glacial acetic acid and is sparingly soluble or insoluble in acetone, dioxane, chloroform, carbon tetrachloride, ethyl acetate, butyl acetate, ethyl ether, petroleum ether, benzene, methyl isobutyl ketone and water; which gives pinkish coloration with concentrated sulfuric acid with subsequently turning brown; which decolorizes a bromine solution and an aqueous potassium permanganate solution; and which shows color reactions,

including negative reaction to biuret, ninhydrin, Molisch, Fehling, ferric chloride, Liebermann-Burchard and 2,4-dinitrophenylhydrazine reagents, but positive reaction to Dragendorff, platinic iodide and Sakaguchi reagents, and a pharmaceutically acceptable salt thereof.

The novel substance of the present invention, which is herein identified as neocopiamycin A, is produced by cultivating the microorganism Streptomyces hygroscopicus var. crystallogenes under controlled aerobic conditions in a culture medium containing sources of carbon and nitrogen, optionally together with the usual essential trace minerals. The parent culture of Streptomyces hygroscopicus var. crystallogenes was isolated from a soil sample taken from a garden of Horyuji Temple, Nara prefecture, Japan. The isolation of the microorganism was carried out by standard dilution procedures employing a culture media which is selective for streptomyces. The organism Streptomyces hygroscopicus var. crystallogenes is a known member of S. hygroscopicus according to the classification of Waksman (The Actinomycetes Vol. II, pp. 143-144, 1961). A careful study of the morphology and physiology of the organism Streptomyces hygroscopicus var. crystallogenes shows it to be distinctly different from any known species and variants of S. hygroscopicus. The term crystallogenes means that a number of small crystals of unidentified nature are formed in the solid medium

around the growth of said organism. From a study of the characteristics of the Streptomyces species which are disclosed in Bergey's Manual of Determinative Bacteriology (8th edition, 1974), Guide to the identification of the Actinomycetes and their Antibiotics, Waksman and Leohevalier, 1953, and The Actinomycetes, Vol. II, Waksman, 1961, it is judged that this strain belongs to the series of hygroscopicus. A culture of Streptomyces hygroscopicus var. crystallogenes was initially deposited in the permanent culture collection of American Type Culture Collection, Rockville, Maryland under the deposit number of ATCC No. 19040 and has newly deposited by the name of Streptomyces hygroscopicus var. crystallogenes IFM 1136 on and since 26th August 1983 in the "Fermentation Research Institute", Agency of Industrial Science and Technology, Ministry of International Trade and Industry of Japan, under deposit number FERM P-7210 and now transmitted and deposited there under FERM BP-576 according to Budapest Treaty.

The microbiological properties of the above-mentioned Streptomyces hygroscopicus var. crystallogenes IMF 1136 will be described below.

(1) Morphology:

On glycerol asparagine agar, the sporophores monopodially branches with numerous closed spirals (sinistrorse) arranged in clusters. Spirals produce in 4 to 6 turns, 20 μ in diameter. Spores, short cylindrical,

1.0 - 2.0 x 1.0 - 1.2 μ, with warty surface structure.

(2) Cultural characteristics:

On sucrose nitrate agar: Growth light yellow (PL10-C-2)* to cinnamon brown (PL12-D-8). Aerial mycelium abundant, powdery, white with gray patches (PL31-A-1) becoming dark gray. Soluble pigment is not produced or slightly brown.

On glucose asparagine agar: Growth spreading, citron to orange (PL10-F-3) later brownish. Aerial mycelium abundant, powdery, slightly yellowish white with brownish gray parts, later becoming moist and black. Soluble pigment is not produced.

On nutrient agar: Growth spreading, minute colonies with elevated centers, light grayish to yellowish brown. Aerial mycelium scant, white. Some parts of colonies remain naked. Soluble pigment is not produced.

On Ca-Malate glycerol agar: Growth spreading, raised colonies, canary yellow (PL11-1-3) to buff (PL-11-K-7). Aerial mycelium abundant, white to greenish yellow (PL18-J-1). Soluble pigment is light greenish yellow.

On yeast extract agar: Growth spreading, raised colonies with depressed centers, brown. Aerial mycelium abundant, dull white to ash gray. Soluble pigment is not produced.

On tyrosine agar: Raised colonies, reddish brown to dark brown. Aerial mycelium abundant, light greenish

yellow. Soluble pigment is faint pink, becoming reddish brown with age.

On potato plug: Growth raised, brown to dull brown. Aerial mycelium white with grayish tinge, later mouse gray. Color of plug is unchanged.

On carrot plug: Growth abundant, raised, light buff. Aerial mycelium abundant, cream-colored to ash gray turning black with age. Soluble pigment is not produced.

On milk: Cream-colored ring is formed. Strong peptonization without coagulation. pH 6.4

On gelatin stab: Growth cream colored to brown. Liquefaction strong. No soluble pigment is formed.

On blood agar: Colonies round, elevated, greenish brown with smooth surface, peripheral parts wrinkled. No aerial mycelium. Soluble pigment is not produced.

(3) Physiological characteristics:

$H_2S$-production: Negative.

Nitrate-reduction: Negative.

Starch is moderately hydrolyzed.

Cellulose is not decomposed.

* Colors shown above are according to the index of "Dictionary of Color" Maery and Paul., McGraw-Hill Book Co., Inc. New York, 1950.

<u>Streptomyces</u> <u>hygroscopicus</u> var. <u>crystallogenes</u> is able to utilize various commercially available sources of

carbon such as glucose, arabinose, fructose, lactose, maltose, sucrose, inositol, raffinose, galactose, rhamnose, xylose, inulin, adonitol, dulcitol, mannitol, sorbitol, sodium succinate and sodium citrate. Sodium acetate does not support the growth of the organism. The test for carbon utilization were carried out according to the procedures of T. C. Pridham and D. Gottlieb (The Utility of Carbon Compounds by some Actinomycetes as an Aid to Species Determination, Journal of Bacteriology, 56, 107-114, (1948)). The organism Streptomyces hygroscopicus var. crystallogenes is capable of growing over a temperature range of 20°C. or less, up to 40°C., but not effectively at 45°C. and above. The main features of S. hygroscopicus var. crystallogenes are as follows:- 1) Formation of closed spirals in aerial mycelium; 2) Warty structure of spore surface; 3) Non-chromogenicity; 4) Lack of significant production of soluble pigment in any culture medium; and 5) Gray coloration of aerial mycelium on most media, which readily moistened turning almost black, clearly indicating that the organism belongs to the Streptomyces hygroscopicus series (Tresner and Backus, A brodened concept of the characteristics of Streptomyces hygroscopicus, Appl. Microbiol., 4, 243-250, 1956). However, when Streptomyces hygroscopicus var. crystallogenes is compared with Streptomyces hygroscopicus CBS (Westerdijk) and S. endus, Gottlieb's strain,

differences in their cultural characteristics and physiological properties are evident, as summarized below.

| Observations | S. hygroscopicus var. crystal-logenes | S. hygro-scopicus | S. endus |
|---|---|---|---|
| Surcrose nitrate agar | | | |
| Growth | light yellow to cinnamon brown | white to light brown | light brown turning dark |
| Ca-malate glycerol agar | | | |
| Growth | carnary yellow | grayish brown | light cream colored |
| Soluble pigment | light greenish yellow | none | none |
| Yeast extract agar | | | |
| Soluble pigment | none | none | slightly brown |
| Carrot plug | | | |
| Aerial mycelium | cream colored | mouse gray | mouse gray |
| Tyrosine agar | | | |
| Soluble pigment | faint pink | none | none |
| Haemolysis | positive | negative | negative |
| Morphology | | | |
| Spirals | 4-6 loops | 4-6 loops | 10 loops |

Such differences are also noticed when the characteristics of Streptomyces hygroscopicus var. crystallogenes are compared with the description of Streptomyces platensis or Streptomyces humidus which are also members of S. hygroscopicus series. Furthermore, Streptomyces hygroscopicus var. crystallogenes forms abundant micro-crystals in the medium around the growth when cultured on yeast extract agar or on malt extract agar. The organism is also characterized by the production of a novel antibiotic neocopiamycin A, which is not known previously.

According to a second aspect of this invention, therefore, there is provided a process for the production of neocopiamycin A, which comprises cultivating a neocopiamycin A-producing strain of Streptomyces hygroscopicus in an aqueous culture medium comprising assimilable carbon and nitrogen sources, under aerobic conditions for a sufficient time to produce and accumulate neocopiamycin A in the culture, and recovering neocopiamycin A from the culture. In the present process, the neocopiamycin A-producing strain may be Streptomyces hygroscopicus var. crystallogenes, now identifiable as FERM P-7210 or FERM BP-576.

It is to be understood that for the production of neocopiamycin A, the present invention is not limited to the use of the aforesaid microorganism Streptomyces

*hygroscopicus* var. *crystallogenes*. It is expressly desired and intended to include the use of mutants which are produced from the described organism by various means such as X-ray radiation, ultraviolet-ray radiation and nitrogen mustards, and also to include the use of any neocopiamycin A-producing microorganisms of this same species, regardless of their origin. The cultivation may be carried out in an aqueous nutrient culture medium under aerobic conditions at a temperature of from about 24 to 35°C. and preferably under submerged conditions of agitation and aeration. Culture media which may be employed for the process include a source of carbon, such as sugares, starch, dextrin, glycerol, and an inorganic or organic source of nitrogen, such as ammonium sulfate, sodium nitrate, ammonium chloride, casein, corn meal, soy bean meal, peanut meal, wheat gluten, cotton seed meal, lactalbumin, enzymatic digests of casein and tryptone. A source of growth substances such as yeast extract, molasses fermentation residues as well as sodium chloride, potassium phosphate, sodium nitrate, magnesium sulfate and trace minerals such as copper, zinc, iron and manganese may also be utilized with desirable results. If excessive foaming is encountered during fermentation, anti-foaming agents such as vegetable oils may be added to the fermentation medium. A buffering agent such as calcium carbonate may also be added to the culture medium.

Inoculum for the preparation of neocopiamycin A by the growth of Streptomyces hygroscopicus var. crystallogenes may be obtained by employing a growth from slants of such media as Emerson's agar or glucose-asparagine agar. The growth or culture may be used to inoculate the sterile culture medium which is placed either in shaken flasks or in inoculum tanks for submerged growth cultivation, or alternatively the inoculum tanks may be seeded from the shaken flasks. The growth of the microorganism usually reaches its maximum in about two or three days, though variations in the equipment used, the rate of aeration, the rate of stirring, etc. may affect the speed with which the maximum potency is reached. In general, the fermentation is continued until substantial amounts of neocopiamycin A are accumulated in the mycelium. Aeration of the culture medium in tanks for submerged cultivation is maintained at the rate of from about one-half to two volumes of free air per volume of the broth per minute.

The production of neocopiamycin A in a very favorable yield may suitably be achieved by the following procedure:- Thus, a sterile culture medium comprising assimilable nitrogen sources such as soybean meal, corn meal, corn steep liquor, fish meat extract, peptone, proteins, protein hydrolysates, amino acids, yeast extract, ammonium sulfate, sodium nitrate, ammonium chloride and the like, and assimilable carbon sources such as glucose, starch,

dextrin, lactose and other assimilable carbohydrates, as well as minor quantities of inorganic salts such as potassium phosphate, magnesium sulfate, potassium chloride, iron sulfate, copper sulfate, zinc sulfate, calcium chloride, manganese chloride, calcium carbonate, sodium chloride and the like is prepared and adjusted to a pH of 6.0 to 8.0. This culture medium is inoculated with a neocopiamycin A-producing strain such as Streptomyces hygroscopicus var. crystallogenes IFM 1136 and then incubated at 27°C under aerobic conditions for a time of 24 to 96 hours sufficient to produce and accumulate neocopiamycin A in the culture. After the completed fermentation, the recovery, isolation and purification of neocopiamycin A may be made as follows. Potency of neocopiamycin A can be estimated according to a dilution assay method or an agar diffusion method using Candida albicans as the assay organism. The active substance usually is present mainly in the mycelium, although it is existing a little also in the liquid phase of the fermentation broth. Therefore, the mycelium cake is separated from the fermentation broth by filtration or centrifugation, washed with water and then extracted with a water-miscible organic solvent such as a lower alkanol, for example, methanol and ethanol. The resulting extract in the organic solvent is concentrated to a smaller volume to deposit a crude material containing the active substance,

with leaving the concentrated solution which still contains an amount of the active substance. When this concentrated solution is adjusted to a lowered pH, the precipitation further proceed, giving a higher amount of the deposited crude material containing the active substance. This deposited material is removed by filtration, washed with water and with acetone and then taken up into a volume of a lower alkanol. The resulting solution in the alkanol is then subjected to a column chromatography on active carbon, silica gel or cellulose as developed with a mixture of chloroform and methanol, ethanol or butanol. The eluate is collected in fractions, and the active fractions are combined together and concentrated under reduced pressure to give a yellowish white colored crude copiamycin complex powder containing both copiamycin and neocopiamycin A.

For isolation and purification of neocopiamycin A, the above-mentioned crude copiamycin complex powder may be subjected to a thin layer chromatography (TLC) on silica gel (for example, Silica Gel 60, Plate $F_{254}$) using a mixture of 2-butanol-water (4:1) as the development solvent, when neocopiamycin A is separated in a single spot at Rf 0.36 while copiamycin is separated in a single spot at Rf 0.25. The region of the single spot at Rf 0.36 is removed from the thin layer chromatographic plate and then extracted with a solvent such as a lower alkanol to give a crude solution of neocopiamycin A,

which may, in turn, be concentrated under reduced pressure to afford a crude powder of neocopiamycin A. When this crude powder of neocopiamycin A is recrystallized from acetone-ethanol (3:1) or from aqueous ethanol, a pure product of neocopiamycin A is obtained as a colorless, crystalline powder.

As far as the inventor has been aware of, the production of neocopiamycin A was achieved efficiently by the following procedures:-

Spores of Streptomyces hygroscopicus var. crystallogenes grown on glucouse-asparagine agar (Krainsky) were inoculated into 500 mℓ Sakaguchi flasks each containing 100 mℓ of a culture medium composed of sources of carbon and nitrogen, as well as the usual essential trace minerals. The flasks were incubated on a reciprocal shaker at 125 stroke per minute, at 27°C. for 48 hours. One litter of the culture broth was transferred into a 200 liter fermentor containing 100 liters of the culture medium as described above. The fermentation was carried out at 27°C. under aeration and agitation. Time course of antibiotic production was followed by a paper disc method using Candida albicans as a test organism. The production of the antibiotics reached maximum after 72 hours of fermentation.

Copiamycin group antibiotics mainly accumulated in the mycelia and were scarcely found in the culture filtrate.

Thus, the mycelial cake was collected from the fermentation broth by continuous centrifugation and washed with water. The collected wet cake was extracted several times with methanol successively. The methanolic extracts were combined and concentrated under reduced pressure. Water was added to the concentrate and the mixture was extracted with 1-butanol. The extract in 1-butanol was evaporated under reduced pressure to give a brown solid. The solid was dissolved in a small volume of methanol and chromatographed on a silica gel column as developing with chloroform, followed by a solvent mixture of chloroform-methanol. Copiamycins were not found in these eluates but were found in those fractions of the eluate which were eluted with chloroform-methanol (3:2) and (1:1). The active fractions of the eluate were combined and evaporated _in vacuo_ to give a slightly yellowish powder. A solution of the powder in methanol, when allowed to stand at room temperature, gave a crystalline precipitate which yield colorless prisms by crystallization from aqueous methanol. The crystalline materials thus obtained were still shown by TLC analysis to be a complex composed of one major component (copiamycin) and several minor including neocopiamycin A. Isolation of neocopiamycin A from the copiamycin complex was achieved by a preparative TLC. The neocopiamycin A-containing portions were scraped out from the TLC plates under

UV light observation, combined and extracted with ethanol. The ethanolic extract waw evaporated under vacuum to remove the solvent, affording a colorless powder of neocopiamycin A.

Referring to the accompanying drawings:-

Figure 1 shows a ultra-violet ray absorption spectrum of neocopiamycin A of this invention in methanol;

Figure 2 shows an infra-red ray absorption spectrum of neocopiamycin A pelleted in potassium bromide; and

Figure 3 shows a nuclear magnetic resonnace (NMR) spectrum of neocopiamycin A.

Neocopiamycin A has the following physico-chemical properties.

(1) Color and Appearance:
neutral, colorless, hygroscopic powder.

(2) Melting point:
134 - 136°C. (dec.)

(3) Specific optical rotation:
$(\alpha)_D^{25}$ = + 23° (c = 1.0, methanol)

(4) Elementary analysis:
Found: C 58.59, H 8.81, N 3.69, O 28.91%
Calcd. for $C_{53}H_{93}O_{17}N_3$; $2H_2O$ (M.W. 1079):
C 58.94, H 8.99, N 3.89, O 28.18%

(5) Molecular weight:
Mass 1,043
FD - MS 1,044 ($MH^+$)

FAB - MS 1,044 ($MH^+$)

SIMS 1,044 ($MH^+$)

(6) Solubility:

Neocopiamycin A is soluble in methanol, ethanol, n-butanol, pyridine, dimethylformamide, glacial acetic acid, but sparingly soluble or insoluble in acetone, dioxane, chloroform, carbon tetrachloride, esters such as ethyl acetate and butyl acetate; petroleum ether, benzene, methyl isobutyl ketone and water.

(7) Color reaction:

The antibiotic neocopiamycin A decolorizes a bromine solution and aqueous potassium permanganate solution. Neocopiamycin A gives negative reactions to biuret, ninhydrin, Molisch, Fehling, ferric chloride, Liebermann-Burchard, 2,4-dinitrophenylhydrazine reagents, but possitive reactions to Dragendorff, platinic iodide and Sakaguchi reagents. It also gives pinkish coloration with conc. sulfuric acid, which subsequently turned brown with time.

(8) Ultra violet absorption spectrum: As shown in Fig. 1 of the accompanying drawings.

$\lambda_{max}^{MeOH}$ : 205 nm (log $\varepsilon$ 4.2)

(9) Infrared absorption spectrum: As shown in Fig. 2 of the accompanying drawing.

IR (KBr)($cm^{-1}$) : 3380, 2975, 2925, 1715, 1655, 1595, 1455, 1375, 1240, 1140, 1070, 975, 845.

(10) NMR spectrum: As shown in Fig. 3 of the accompanying drawing.

(11) Rf values of TLC:

A purified sample of neocopiamycin A gives only one spot when chromatographed on thin layer chromatography TLC (Silica gel 60, plate $F_{254}$) in different solvent systems and bioautographed against Candida albicans or absorption of ultraviolet. The Rf values and solvent systems employed are summarized in Table 1.

Table 1: Rf values of neocopiamycin A in several solvent systems.

| Solvent system | Rf |
|---|---|
| 2-butanol-water (4:1) | 0.36 |
| n-butanol-acetic acid -water (67:23:10) | 0.33 |
| acetone-methanol (3:2) | 0.38 |

(12) Chemical structure:

According to spectral data and physicochemical characteristics, the sturcture of neocopiamycin A can be presumed to have the following formula:-

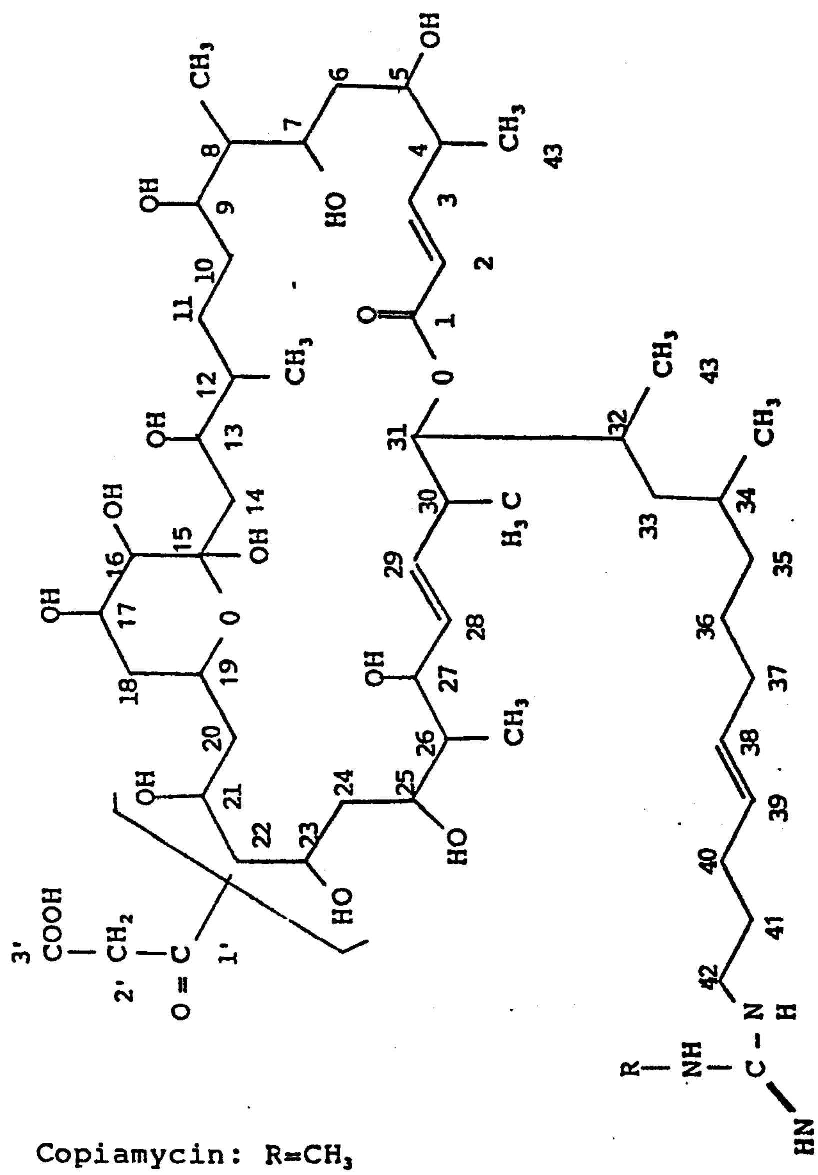

Copiamycin: R=$CH_3$

Neocopiamycin A: R=H

Neocopiamycin A has the following biological properties.

(13) Antimicrobial Activity

The minimum inhibitory concentration (MIC) of neocopiamycin A against bacteria, yeast and fungi were determined by a serial two-fold agar dilution method. The medium used was Muller-Hilton agar for bacteria, and Sabouraud dextrose agar for yeasts and fungi. The antibiotic was dissolved in a small amount of ethanol and diluted with sterilized water to give final agar media containing less than one percent of the organic solvent, which was not a harmful concentration against the organisms tested. The MICs were determined after incubation for 24 hours or 4 days at 37°C for bacteria, and after incubation 24 hours to 7 days at 37°C for yeasts and fungi, depending on the test strain.

Antimicrobial spectrum of neocopiamycin A is given in Tables 2, 3 and 4 below. The tables 2, 3 and 4 show that neocopiamycin A is active against a wide range of fungi, yeasts and Gram-positive bacteria but inactive against Gram-negative bacteria, and that it has much higher antimicrobial activity than copiamycin. Furthermore, it should be pointed out that neocopiamycin A inhibits the growth of both the filamentous and yeast-like fungi at the concentrations below 10 μ g/ml. The growth of Trichomonas vaginalis was inhibited at the

concentration of 1.56 μg/ml of neocopiamycin A in thioglycolate medium with 0.5% dextrin and 15% bovine serum. Antifungal spectra of copiamycin were similarly determined for the comparison purpose.

Table 2: Antifungal spectra of neocopiamycin A and copiamycin (comparative)

| Test organisms | MIC (mcg/ml) | |
|---|---|---|
| | Neocopiamycin A | Copiamycin |
| Aspergillus flavus 23 | 6.25 | 100 |
| A. fumigatus 25 | 25.0 | >100 |
| A. nidulans 21 | 6.25 | 100 |
| A. niger 22 | 6.25 | >100 |
| A. oryzae IFM 40607 | 6.25 | >100 |
| A. versicolor 26 | 12.5 | >100 |
| Penicillium expansum IFM 40619 | 3.13 | 100 |
| Epidermophyton floccosum IFM 40747 | 0.78 | 0.39 |
| Microsporum canis | 1.56 | 3.13 |
| M. gypseum IFM 40727 | 1.56 | 6.25 |
| Trichophyton mentagrophytes IFM 40737 | 1.56 | 3.13 |
| T. mentagrophytes Kamiyama | 1.56 | 3.13 |
| T. rubrum IFM 40732 | 0.39 | 3.13 |
| Sporothrix schenckii IFM 40750 (hyphal phase) | 6.25 | 50 |
| Fonsecaea pedrosoi IFM 40756 | 3.13 | 100 |
| Histoplasma capsulatum IFM 40752 (hyphal phase) | 0.78 | 0.78 |

Table 3: Antigungal (anti-yeast) spectra of neocopiamycin A and copiamycin (comparative)

| Test organism | MIC (mcg/ml) | |
|---|---|---|
| | Neocopiamycin A | Copiamycin |
| Candida albicans IFM 40001 | 1.56 | 12.5 |
| C. albicans IFM 40002 | 3.13 | 25 |
| C. albicans IFM 40003 | 3.13 | 25 |
| C. albicans IFM 40004 | 6.25 | 100 |
| C. albicans IFM 40005 | 1.56 | 25 |
| C. albicans IFM 40007 | 3.13 | 12.5 |
| C. albicans IFM 40008 | 1.56 | 12.5 |
| C. albicans IFM 40009 (7N) | 6.25 | 100 |
| C. guilliermondii IFM 40017 | 3.13 | 100 |
| C. tropicalis IFM 40018 | 3.13 | 100 |
| C. Krusei IFM 40019 | 3.13 | 100 |
| C. parapsilosis IFM 40020 | 3.13 | 100 |
| C. stellatoidea IFM 40021 | 3.13 | 100 |
| C. utilis IFM 40099 | 12.5 | >100 |
| Cryptococcus neoformans IFM 40037 | <0.78 | 1.56 |
| C. neoformans IFM 40038 | <0.78 | 1.56 |
| C. neoformans IFM 40047 | 1.56 | 6.25 |
| Geotrichum candidum IFM 40068 | 1.56 | 1.56 |
| Torulopsis glabrata IFM 40065 | 6.25 | 100 |
| Trichosporon cutaneum IFM 40066 | 6.25 | 100 |
| Saccharomyces cerevisiae sake IFM 40025 | 12.5 | >100 |
| Sporothrix schenckii IFM 40751 (yeast phase) | 3.13 | 100 |

Table 4: Antibacterial spectra of neocopiamycin A.

| Test organism | MIC (mcg/ml) |
|---|---|
| Bacillus subtilis PCI 219 | 12.5 |
| Micrococcus luteus IFM 2066 | 6.25 |
| Staphylococcus aureus 209P | 12.5 |
| S. albus IFM 2013 | 12.5 |
| S. citreus IFM 2025 | 12.5 |
| Streptococcus faecalis IFM 2001 | 100.0 |
| Corynebacterium diphteriae IFM 2056 | 3.13 |
| Escherichia coli | >100.0 |
| Klebsiella pneumoniae IFM 3008 | 25.0 |
| Proteus vulgaris IFM 3014 | >100.0 |
| Pseudomonas aeruginosa IFM 3011 | >100.0 |
| Salmonella typhimurium IFM 3023 | >100.0 |
| Serratia marcescens IFM 3027 | >100.0 |
| Mycobacterium sp. 607 | >100.0 |
| Nocardia asteroides IFM 0042 | 25.0 |
| N. brasiliensis IFM 0082 | 25.0 |
| Streptomyces paraguayensis IFM 1148 | 50.0 |

(14) Acute Toxicity

Acute toxicity of neocopiamycin A was determined with ddY mice weighing 20 to 21 g. Neocopiamycin A was finely pulverized and suspended in 15% aqueous ethanol solution for intraveneous, intraperitoneal and oral administrations. Neocopiamycin A was relatively nontoxic as follows: All mice tolerated for 14 days without any toxic signs, upon intravenous injection of 30 mg and upon intraperitoneal and oral administrations of more than 1,000 mg/kg of neocopiamycin A.

As already described, neocopiamycin A of this invention possesses a high antimicrobial activity against a wide variety of fungi, yeasts and Gram-positive bacteria. Further, neocopiamycin A has a low toxicity to animals as shown by the acute toxicity tests shown above. Thus, neocopiamycin A of this invention is very useful as an antifungal agent and for this purpose it is generally formulated into the form of pharmaceutical composition, which may be administered to man or an animal in a way known per se.

Accordingly, this invention also provides a pharmaceutical composition comprising as the active ingredient a therapeutically or antifungally effective amount of neocopiamycin A or a pharmaceutically acceptable salt thereof,in association with a pharmaceutically acceptable carrier or adjuvant which may be conventional one, such

as those used for the preparations of copiamycin. The pharmaceutical composition according to this invention may further comprises a therapeutically or antifungally effective amount of copiamycin, if desired. The composition may contain neocopiamycin A at a concentration of e.g., 0.1% to 10% based on the weight of the composition. The composition may suitably be formulated into conventional preparations for external application, for example, by admixing with a liquid carrier such as ethanol or a semi-solid carrier such as polyethylene glycol.

This invention further provides a method for inhibiting the growth of fungi, yeasts and/or bacteria, which comprises contacting an inhibitingly effective amount of neocopiamycin A with the fungi, yeasts and/or bacteria. This invention also provides a method for therapeutically treating infection by fungi, yeasts and/or bacteria in a living animal, including human beings, which comprises administering to the animal a therapeutically effective amount of neocopiamycin A. It will be appreciated that an appropriate effective amount of the effective ingredient to be administered for the envisaged purpose will vary depending upon the particular composition formulated, the mode of administration, the conditions to be treated and the nature of the fungi, yeasts or bacteria to be controlled thereby.

This invention is further illustrated but not limited by the following Examples.

Example 1

Production of neocopiamycin A by shake cultivation.

The microorganism, Streptomyces hygroscopicus var. crystallogenes IFM 1136 (FERM P-7210; FERM BP-576) was cultured on surface of Yeast - Starch - Agar slant (comprising yeast extract 0.1%, soluble starch 0.5%, agar 1.5%, and tap water 1,000 ml, pH 7.0) at 25°C for 2 weeks. Well sporulated slant was used at an inoculum size of two loopful amounts to inoculate 100 ml of a culture medium having the following composition:

| | |
|---|---|
| Meat extract | 0.5% |
| Polypepton | 1.0% |
| Glucose | 0.1% |
| Soluble starch | 1.0% |
| NaCl | 0.3% |
| Tap water | 1,000 ml. |
| pH | 7.0 - 6.8 |

which had been sterilized by autoclaving at 15 pounds steam pressure at 120°C for 30 minutes. One hundred 500 ml - shake flasks containing 100 ml of the above culture medium which was inoculated from the agar slants were incubated at 27°C for 72 hours on a reciprocal shaker.

At the end of this incubation period, the cultures were combined together and the mycelial cake was harvested

by filtration from the culture broth. The mycelial cake was repeatedly washed with water and the resulting 700 g of the wet mycelium was extracted with 2 l. of methanol. The methanolic extract was taken by filtration and the mycelium residue was again extracted with an equal volume of methanol, and with 2 l. of methanol. All the methanolic extracts so obtained were combined and concentrated in vacuo at a temperature below 60°C to one-tenth its original volume. The brown concentrated solution was extracted with n-butanol and was again concentrated in vacuo. Forty nine grams of the resulting crude residue was washed with 100 ml of water and dried to give 24 g of the crude powder. The powder was dissolved in a small volume of methanol and chromatographed on a silica gel column (Silica Gel 60, 70-130 meshes, Mallincrodt Co., U.S.A.), as developed with chloroform, followed by a solvent mixture of chloroform-methanol (3:2). The active fractions of the eluate were combined and evaporated in vacuo to give 2.5 g of slightly yellowish powder. After washing this powder with water and methanol, one gram of a white powder containing copiamycin, neocopiamycin A and other antibiotics was obtained.

Isolation of neocopiamycin A from said white powder comprising the copiamycin complex was achieved by TLC on silica gel 60 $F_{254}$ (20cm x 20cm, 0.5mm thick) using 2-butanol-water (4:1) as the developing solvent. The crude neocopiamycin A appeared as one minor component (at

Rf 0.36) by TLC analysis. The neocopiamycin A - containing portions were scraped out from TLC plates under UV light observation and were combined, followed by extracting with ethanol. The ethanolic extract was evaporated under vacuum to afford a white powder comprising a crude neocopiamycin A (0.63 g). Recrystallization of this powder from acetone-ethanol (3:1) and again from aqueous ethanol gave a colorless powder of pure neocopiamycin A (0.45 g). m.p. 134-136°C (dec.).

Example 2

Production of neocopiamycin A by tank cultivation.

Ten 500 ml-capacity Sakaguchi flasks each containing 100 ml of the culture medium having the composition as described in Example 1 were inoculated in the same manner as in Example 1 with the yeast-Starch-Agar slant cultures of the strain IFM 1136 which had been cultured at 25°C for two weeks. The flasks so inoculated were incubated at 25°C for two days as in Example 1 on a rotary shaker (280 r.p.m.) to give a seed culture ( 1 l.). This seed culture was then introduced at 10% inoculum into a 200 l.-fermentation tank containing 100 l. of the same culture medium. Aeration were maintained at one-half of the medium volume and agitation was made at 250 r.p.m. The incubation was continued at 27°C for 72 hours and under an inner pressure of 0.5 kg/cm. After the incubation, 7.8 kg of the wet mycelium was harvested by continuous centrifugation (10,000 r.p.m.)

and washing with water. The mycelial cake was extracted three times with 10 l. portions of methanol, respectively. The methanol extractes was combined to a 64 l. volume and concentrated to a 6 l. volume in a continuous flash evaporator. An equal volume of water was added to the resulting concentrate, followed by addition of sodium chloride, and the mixture was extracted three times with an equal volume of 1-butanol. The extract was filtrated and concentrated under reduced pressure to give a crude material (480 g). This crude material was washed with 1 l. of water and dried to give a crude powder (250 g). The solid (the powder) was dissolved in a small volume of methanol and chromatographed on a silica gel column as developed with chroloform, followed by development with a solvent mixture of chloroform-methanol (3:2). The active fractions of the eluate were combined and evaporated in vacuo to give a slightly yellowish powder (110 g). This yellowish powder was washed with methanol and then with water to afford a white powder (43 g). A methanolic solution of the white powder, when allowed to stand at room temperature, gave a crystalline precipitate which in turn, yielded colorless prisms (23 g) by recrystallization from aqueous methanol. The prism-crystalline material thus obtained were shown to be a complex composed of one major component (copiamyin, Rf 0.25) and several minor components including neocopiamycin A (Rf 0.36), by a

TLC analysis (Silica gel 60 F, 20cm x 20cm, 0.5mm thick, Merk; solvent system: 2-butanol-water, 4:1; detection: UV light). The neocopiamycin A-containing materials (at Rf 0.36) were scraped out from the TLC plates, then were combined and extracted with methanol. The solvent was evaporated off from the extract under vacuum to give a white powder. Recrystallization of this white powder from acetone-ethanol (3:1) and again from aqueous ethanol gave a colorless powder of pure neocopiamycin A (1.6 g).

CLAIMS:-

1. A new antibiotic, neocopiamycin A, which is in the form of a colorless, hygroscopic substance having a melting point of 134-136°C (dec.) and giving a molecular formula $C_{53}H_{93}O_{17}N_3$; which is soluble in a lower alkanol including methanol, ethanol and n-butanol, pyridine, dimethylformamide and glacial acetic acid and is sparingly soluble or insoluble in acetone, dioxane, chloroform, carbon tetrachloride, ethyl acetate, butyl acetate, ethyl ether, petroleum ether, benzene, methyl isobutyl ketone and water; which gives pinkish coloration with concentrated sulfuric acid with subsequently turning brown; which decolorizes a bromine solution and an aqueous potassium permanganate solution; and which shows color reactions, including negative reaction to biuret, ninhydrin, Molish, Fehling, ferric chloride, Liebermann-Burchard and 2,4-dinitrophenylhydrazine reagents, but positive reaction to Dragendorff, platinic iodide and Sakaguchi reagents, and a pharmaceutically acceptable salt thereof.

2. A process for the production of neocopiamycin A as claimed in Claim 1, which comprises cultivating a neocopiamycin A-producing strain of Streptomyces hygroscopicus in an aqueous culture medium comprising assimilable carbon and nitrogen sources, under aerobic conditions for a sufficient time to produce and accumulate neocopiamycin A in the culture, and recovering neocopiamycin A from the culture.

3. A process according to Claim 2 in which the neocopiamycin A-producing strain is Streptomyces hygroscopicus var. crystallogenes identified as FERM-BP 576.

4. A process according to Claim 2 in which the cultivation of the neocopiamycin A-producing strain is carried out at a temperature of 24°C to 35°C, preferably 27°C for about 2 days to about 5 days.

5. A pharmaceutical composition comprising as the active ingredient a therapeutically or antifungally effective amount of neocopiamycin A, in association with a pharmaceutically acceptable carrier.

6. A pharmaceutical composition according to Claim 5 which further comprises a therapeutically or antifungally effective amount of copiamycin.

7. A method for inhibiting the growth of fungi, yeasts and/or bacteria, which comprises contacting an inhibitingly effective amount of neocopiamycin A with fungi, yeasts and/or bacteria.

FIG. 1

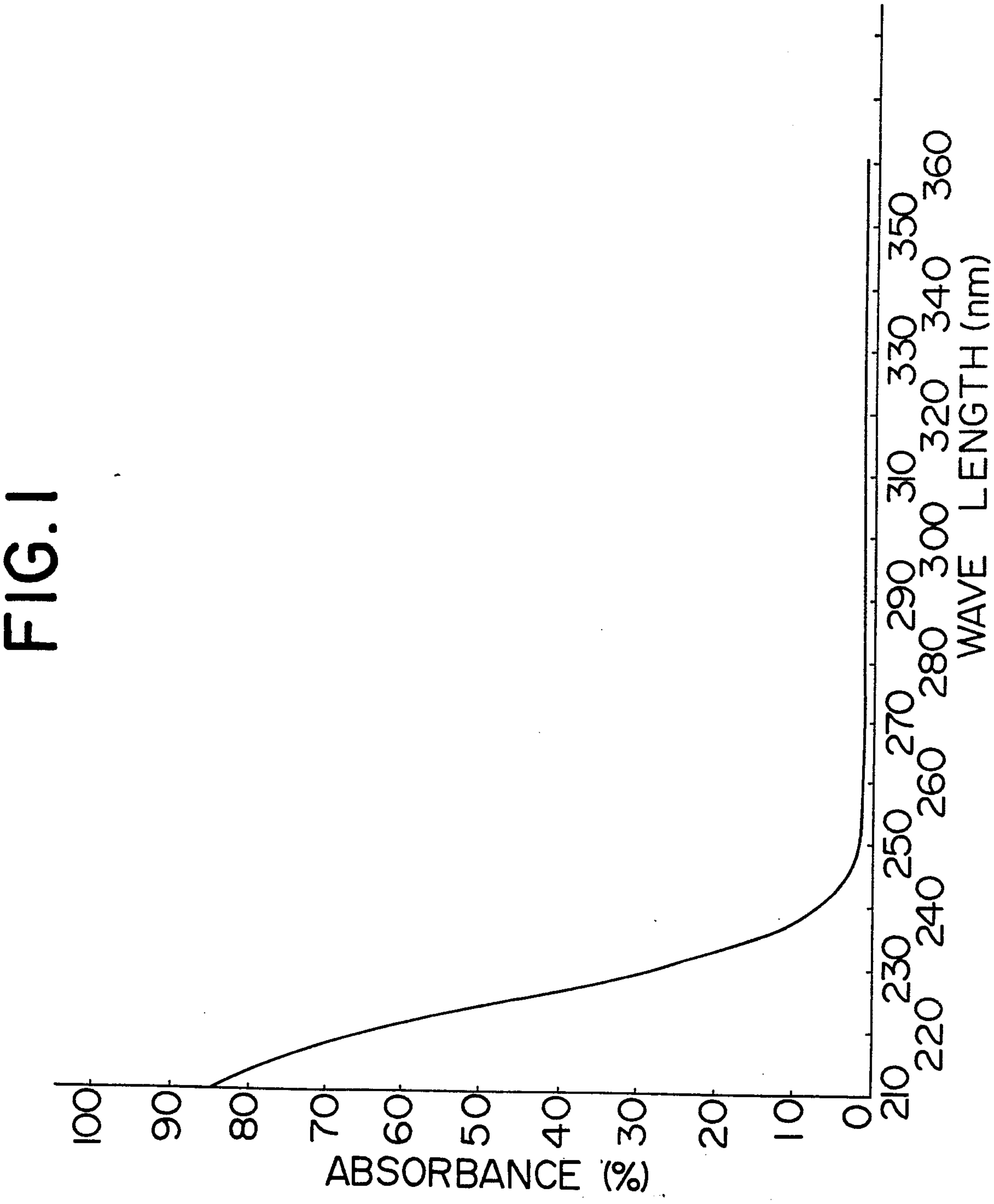
ABSORBANCE (%)
100
90
80
70
60
50
40
30
20
10
0
210
220
230
240
250
260
270
280
290
300
310
320
330
340
350
360
WAVE LENGTH (nm)

FIG. 2

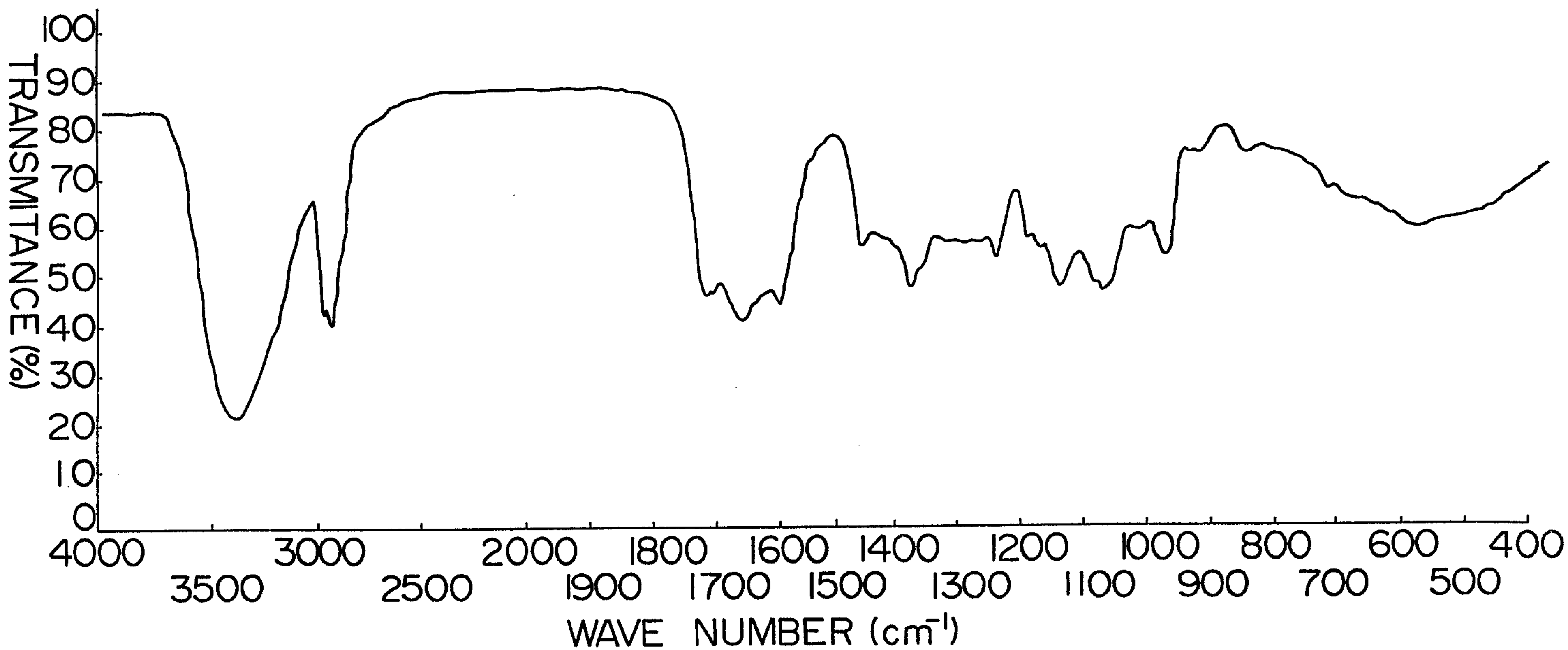

TRANSMITANCE (%)
100
90
80
70
60
50
40
30
20
10
0
4000
3500
3000
2500
2000
1900
1800
1700
1600
1500
1400
1300
1200
1100
1000
900
800
700
600
500
400
WAVE NUMBER (cm⁻¹)

0137692

3/3

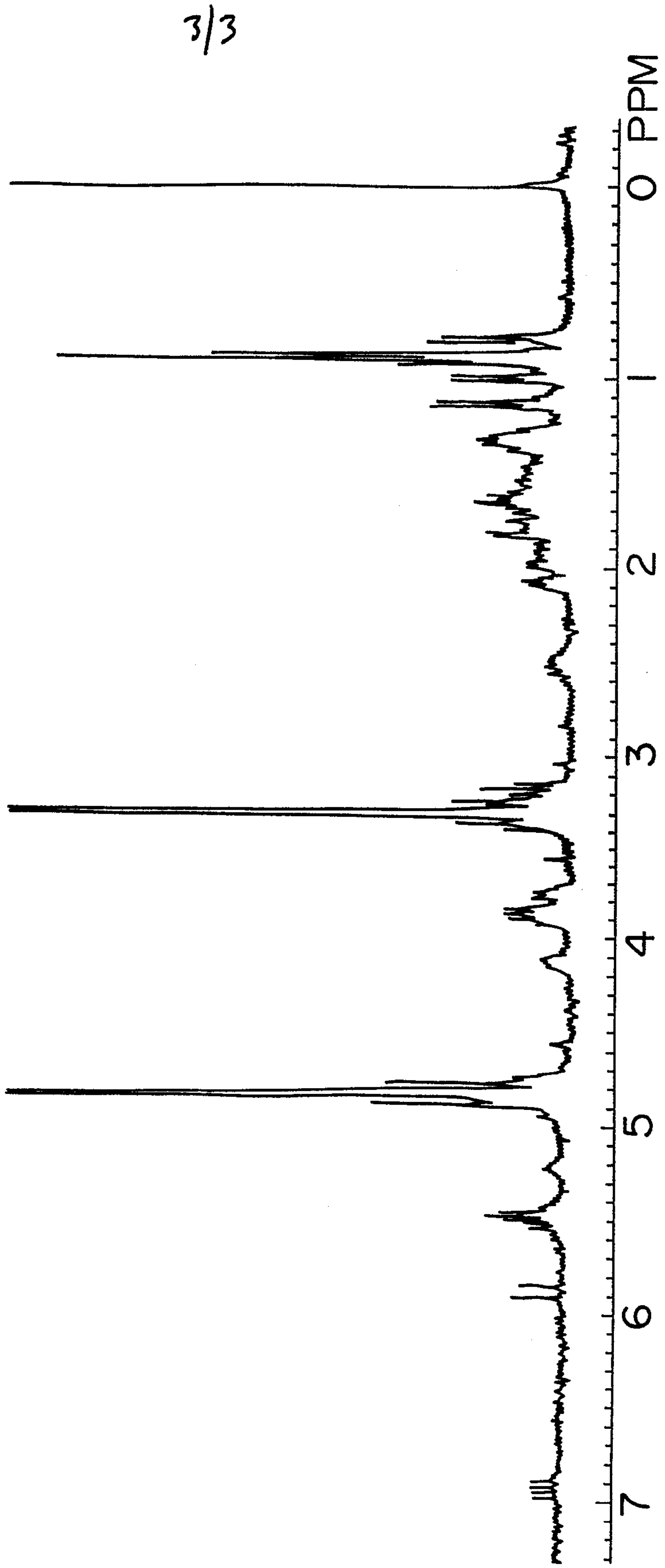


FIG. 3